# EUROPÄISCHE PATENTSCHRIFT

(11) **EP 3 191 013 B1**
(45) Veröffentlichungstag und Bekanntmachung des Hinweises auf die Patenterteilung: **17.03.2021**
(21) Anmeldenummer: 15745360.6
(22) Anmeldetag: 01.07.2015
(51) Int. Cl.: A61C 13/00, C04B 35/64, F27D 5/00

(54) **SINTERROHLING**
SINTER BLANK
ÉBAUCHE DESTINÉ AU FRITTAGE

(30) Priorität: 12.09.2014 DE 102014113148
(43) Veröffentlichungstag der Anmeldung: 19.07.2017
(73) Patentinhaber: Amann Girrbach AG, 6842 Koblach (AT)
(72) Erfinder: REICHERT, Axel, 9443 Widnau (CH)
(74) Vertreter: Fechner, Thomas
(86) Internationale Anmeldenummer: PCT/AT2015/000095
(87) Internationale Veröffentlichungsnummer: WO 2016/037199

(56) Entgegenhaltungen:
- EP-A1- 2 014 254
- EP-A1- 2 602 036
- EP-A1- 2 792 985
- WO-A1-2012/064257
- DE-A1-102009 044 461
- JP-U- S60 102 006

## Beschreibung

Die vorliegende Erfindung betrifft einen Sinterrohling zur Herstellung einer Dentalprothese mit zumindest einem Sintervorgang, wobei der Sinterrohling zumindest einen Produktbereich, aus dem die Dentalprothese entsteht, und zumindest eine Verstrebung, welche nach dem Sintervorgang zu entfernen ist, aufweist. Weiters betrifft die Erfindung auch eine Sinterkammer.

Zur Herstellung einer Dentalprothese werden heutzutage häufig formgebend bearbeitete Sinterrohlinge einem Sintervorgang unterzogen, sodass im Ergebnis des Sintervorgangs gegebenenfalls mit noch weiteren Nachbearbeitungsschritten die Dentalprothese aus dem Sinterrohling oder Teilen davon entsteht. Die Sinterrohlinge können dabei beim Stand der Technik sowohl aus keramischen als auch metallischen Werkstoffen bestehen. Beim Sintervorgang selbst kommt es praktisch immer zu einem Schrumpfprozess. Weiters handelt es sich bei dem noch nicht gesinterten Material, also beim Sinterrohling, um ein nicht sehr stabiles Material. Die endgültige Materialfestigkeit wird erst durch zumindest einen Sintervorgang erreicht. Die Sintertemperatur liegen dabei relativ nahe am Schmelzpunkt des Materials, sodass der zu sinternde Formkörper bzw. Sinterrohling bis zum Abschluß des Sintervorgangs sehr empfindlich ist.

Die Praxis zeigt, dass es vor allem bei größeren Sinterrohlingen bzw. bei daraus herzustellenden Dentalprothesen schwierig ist, einen verzugsfreien Sintervorgang sicherzustellen. Es kommt oft zu ungewollten Verformungen durch Verzug. Es wurde daher bereits in der Praxis versucht, Verstrebungen in den Sinterrohlingen zu integrieren, um den Produktbereich, also den Teil des Sinterrohlings aus dem die spätere Dentalprothese entsteht, abzustützen.

In der EP 2 014 254 A1 und der DE 10 2009 044 461 A sind gattungsgemäße Sinterrohlinge gezeigt. In der EP 2 014 254 A1 ergeben sich zwischen der Verstrebung und einer Basisfläche, auf der die Verstrebung abgestellt wird, letztendlich linienförmige Kontaktkanten. Die DE 10 2009 044 461 A1 lehrt an der Verstrebung eine ebenförmige Begrenzungsfläche.

Aufgabe der Erfindung ist es, Sinterrohlinge der oben genannten Art dahingehend weiter zu verbessern, dass ein möglichst verzugsfreies Sintern des Sinterrohlings möglich ist.

Dies wird erfindungsgemäß durch einen Sinterrohling gemäß Patentanspruch 1 erreicht.

Es ist somit vorgesehen, dass die Verstrebung zumindest eine Gleitnoppe zum Abstützen der Verstrebung auf einer Basisfläche während des Sintervorgangs aufweist.

Durch die zumindest eine erfindungsgemäße Gleitnoppe an der Verstrebung wird einerseits erreicht, dass die Verstrebung selbst auf der Basisfläche während des Sintervorgangs abgestützt ist. Hierdurch lastet das Eigengewicht der Verstrebung nicht auf dem Produktbereich des Sinterrohlings. Darüber hinaus kann die Gleitnoppe beim sinterbedingten Schrumpfen des Sinterrohlings über die Basisfläche gleiten, um so ein möglichst verzugsfreies Schrumpfen des Sinterrohlings zu ermöglichen. Durch die Gleitnoppe wird der Gleit- und Haftreibungswiderstand der Verstrebung, verglichen mit dem Fall, dass die Verstrebung vollflächig auf der Basisfläche aufliegt, stark reduziert.

Der Sinterrohling wird häufig auch als Grünling bezeichnet. Er besteht meist aus verdichtetem Ausgangsmaterial bzw. Pulver. Erfindungsgemäße Sinterrohlinge können sowohl aus keramischen als auch metallischen Werkstoffen bestehen bzw. hergestellt werden. Im Sinterrohling sind Produktbereich und Verstrebung sowie gegebenenfalls vorhandene weitere Bauteile in der Regel einstückig miteinander verbunden. In der Regel bestehen alle Teile des Sinterrohlings aus demselben Ausgangsmaterial bzw. derselben Zusammensetzung des Ausgangsmaterials. Beim Sinterrohling kann es sich um einen Rohling handeln, welcher noch vollständig ungesintert ist. Es kann aber auch sein, dass der Sinterrohling bereits teilweise gesintert bzw. vorgesintert ist und in dem Sintervorgang oder noch weiteren Sintervorgängen noch fertig gesintert werden muss. Der Produktbereich des Sinterrohlings ist der Teil des Sinterrohlings, aus welchem später die Dentalprothese entsteht. Der Produktbereich weist in der Regel bereits eine ähnliche Form wie die Dentalprothese auf. Da beim Sintern ein Schrumpfen des Materials zu berücksichtigen ist, ist der Produktbereich aber in der Regel entsprechend größer dimensioniert als die fertig gesinterte Dentalprothese. Außerdem kann die Dentalprothese insbesondere nach dem Sintern noch einer weiteren Nachbearbeitung, z.B. zur Entfernung der Verstrebung und/oder der nachfolgend noch genannten Standfüße, unterzogen werden, wodurch sich wiederum Unterschiede in der Form zwischen Produktbereich und Dentalprothese ergeben können. Die Verstrebung dient ausschließlich der Abstützung bzw. Versteifung bzw. Verstrebung des Produktbereichs während des Sintervorgangs. Die Verstrebung selbst ist am Endprodukt, also an der fertigen Dentalprothese nicht mehr zu sehen, da sie nach dem Sintervorgang vom Produktbereich abgetrennt, abgeschliffen oder auf andere Art und Weise entfernt wird. Jedenfalls ist es so, dass der Produktbereich und die Verstrebung mit der zumindest einen Gleitnoppe im Sinterrohling bereits ausgeformt und damit als solche auch optisch erkennbar sind. Der Begriff der Dentalprothesen umfasst grundsätzlich alle künstlich angefertigten Teile, welche die entsprechenden natürlichen Teile eines Gebisses im Mund des Patienten ersetzen. Es kann sich somit um künstliche Zähne, Teile von künstlichen Zähnen, vor allem aber auch um Zahnbögen, Brücken und dergleichen handeln. In diesem Zusammenhang sehen besonders bevorzugte Ausgestaltungsformen der Erfindung vor, dass der Produktbereich ein Zahnbogen mit mehreren, vorzugsweise paarweise, untereinander verbundenen Zähnen ist und die Verstrebung zumindest zwei Zähne dieses Zahnbogens miteinander verbindet. Besonders bevorzugt ist dabei vorgesehen, dass der Zahnbogen einen Innenraum teilweise umschließt und die Verstrebung zumindest teilweise im Innenraum angeordnet ist. Bei Dentalprothesen kann es sich aber auch um Hilfseinrichtungen handeln, die der Herstellung oder Befestigung von künstlichen Zähnen, Brücken, Zahnbögen oder dergleichen im Mund des Patienten dienen. In diesem Zusammenhang ist darauf hinzuweisen, dass, wenn hier von Zähnen bzw. Zahnbögen gesprochen wird, es sich, soweit sich nichts anderes aus dem Zusammenhang ergibt, um künstliche Zähne bzw. Zahnbögen handelt.

Der Begriff der Gleitnoppe bezeichnet eine Erhebung auf einer entsprechenden Oberfläche der Verstrebung, mit welcher die Verstrebung besser auf einer Basisfläche während des Sintervorgangs gleiten kann, sodass im Produktbereich und in der Verstrebung auftretende Schrumpfvorgänge nicht zu einem Verzug führen.

Die Gleitnoppe und auch ihre nachfolgend noch genannte Auflagefläche können sehr unterschiedliche Formen und Größen aufweisen, z.B. stab- oder kegel- oder halbkugelförmig ausgebildet sein. Die Basisfläche ist die Fläche, auf welcher der Sinterrohling während des Sintervorgangs abgestützt ist.

Insbesondere bei größeren Verstrebungen ist in bevorzugten Ausgestaltungsformen der Erfindung vorgesehen, dass die Verstrebung mehrere zueinander distanziert angeordnete Gleitnoppen zum Abstützen der Verstrebung auf der Basisfläche während des Sintervorgangs aufweist. Der Begriff "mehrere" umfasst dabei, wie er auch weiter unten verwendet wird, grundsätzlich eine Anzahl von zwei und mehr.

Um die Reibung gegenüber der Basisfläche möglichst gering zu halten, ist vorgesehen, dass die bzw. jede einzelne Gleitnoppe zum Abstützen der Verstrebung auf der Basisfläche während des Sintervorgangs eine gegenüber der Verstrebung reduzierte Auflagefläche aufweist. Gegenüber der Verstrebung reduzierte Auflagefläche bedeutet dabei, dass die Auflagefläche der Gleitnoppe, also die Fläche, mit der die Gleitnoppe in der Betriebsstellung also beim Sintern auf der Basisfläche aufliegt, kleiner ist als die Fläche, mit der die Verstrebung in der Betriebsstellung auf der Basisfläche aufliegen würde, wenn keine Gleitnoppe vorhanden wäre. Die Auflagefläche der Gleitnoppe(n) ist in bevorzugten Ausführungsformen möglichst klein. Bevorzugt ist die Auflagefläche der Gleitnoppe(n) also, vorzugsweise jeweils, auf einen Auflagepunkt reduziert. Von einem Auflagepunkt spricht man, wenn die Auflagefläche so klein ist, dass sie dem bloßen menschlichen Auge als Punkt erscheint. Für den Fall dass mehrere Gleitnoppen an der Verstrebung vorhanden sind, gilt bevorzugt, dass die, vorzugsweise jede der, Gleitnoppen zum Abstützen der Verstrebung auf der Basisfläche während des Sintervorgangs jeweils eine gegenüber der Verstrebung reduzierte Auflagefläche, vorzugsweise einen Auflagepunkt, aufweisen. Dies gilt günstigerweise auch für die Summe aller Auflageflächen aller Gleitnoppen auf einer Verstrebung. Auch die Summe dieser Auflageflächen ist bevorzugt kleiner als die Fläche, mit der die Verstrebung in der Betriebsstellung auf der Basisfläche aufliegen würde, wenn keine Gleitnoppen vorhanden wären.

Beim Sintern bedingt der am Sinterrohling auftretende Schrumpfprozess, dass verschiedene Teile und Bereiche des Sinterrohlings in voneinander verschiedene Richtungen bewegt werden. Um ein Abgleiten der Gleitnoppe in alle Richtungen gleich gut zu gewährleisten und somit Schrumpfungsprozesse in allen Richtungen gleich gut ausgleichen zu können, sehen bevorzugte Ausgestaltungsformen der Erfindung vor, dass die Auflagefläche der Gleitnoppe ein Auflagepunkt ist. Ist dies nicht der Fall, so ist es günstig, wenn die Auflagefläche der Gleitnoppe eine maximale Breite und eine maximale Länge aufweist, welche möglichst gleich groß sind. Günstigerweise weicht die maximale Breite der Auflagefläche um höchstens 50% von der maximalen Länge der Auflagefläche ab. Besonders günstig ist in diesem Zusammenhang eine kreisförmige oder ringförmige Auflagefläche. Die Auflagefläche kann aber auch andere Formen wie z.B. eine quadratische oder dreieckige Form oder dergleichen haben. Der Auflagepunkt bzw. die Auflagefläche des Gleitnoppens ist jedenfalls der Bereich des Gleitnoppens, mit dem der Gleitnoppen in der Betriebsstellung auf der Basisfläche aufliegt.

Um auch den Produktbereich möglichst gut auf der Basisfläche abstützen zu können, sehen bevorzugte Ausgestaltungsformen der Erfindung vor, dass der Sinterrohling am Produktbereich einen Standfuß oder mehrere voneinander distanziert angeordnete Standfüße zum Abstützen des Produktbereichs auf der Basisfläche während des Sintervorgangs aufweist. Da die Standfüße vom Produktbereich nach dem Sintervorgang in der Regel durch Schleifen entfernt werden müssen, sind sie günstigerweise stiftförmig ausgebildet. Sie können aber genauso wie die Gleitnoppen z.B. auch halbkugelförmig oder kegelförmig ausgebildet sein. Damit auch der Standfuß bzw. die Standfüße beim Sintervorgang mit möglichst geringem Widerstand über die Basisfläche gleiten können, sehen bevorzugte Ausgestaltungsformen der Erfindung vor, dass der Standfuß oder die Standfüße zum Abstützen des Produktbereichs auf der Basisfläche während des Sintervorgangs, vorzugsweise jeweils, eine gegenüber dem Produktbereich reduzierte Auflagefläche, vorzugsweise einen Auflagepunkt, aufweist bzw.
aufweisen. Die punktförmigen bzw. kreisförmigen Auflageflächen der Standfüße dienen hier demselben Ziel wie bei den Gleitnoppen. Zu Ihrer Form und Größe gilt somit auch das oben zu den Auflageflächen der Gleitnoppen Gesagte.

Bevorzugte Ausgestaltungsformen sehen vor, dass die Basisfläche eine Ebene ist. Besonders bevorzugt ist vorgesehen, dass alle Auflagepunkte und/oder Auflageflächen aller Gleitnoppen und soweit vorhanden auch aller Standfüße in einer gemeinsamen Ebene angeordnet sind.

Um das Entlanggleiten der Gleitnoppen und gegebenenfalls auch der Standfüße während des sinterbedingten Schrumpfvorgangs des Sinterrohlings möglichst reibungsfrei zu gestalten, sieht die Erfindung auch eine Sinterkammer zum Sintern zumindest eines erfindungsgemäßen Sinterrohlings vor, wobei die Sinterkammer eine ebene Gleitfläche als Basisfläche zum Abstellen des Sinterrohlings auf dieser Basisfläche während des Sintervorgangs aufweist. Gemäß der Erfindung handelt es sich dabei um eine Sinterkammer mit zumindest einem erfindungsgemäßen Sinterrohling. Es handelt sich sozusagen um eine Anordnung mit einer Sinterkammer und zumindest einem erfindungsgemäßen Sinterrohling, wobei die Sinterkammer eben eine ebene Gleitfläche als Basisfläche zum Abstellen des Sinterrohlings auf dieser Basisfläche während des Sintervorgangs aufweist. Der Sinterrohling ist dabei während des Sintervorgangs günstigerweise auf dieser Basisfläche abgestellt. Die Basisfläche kann ein separates Bauteil, wie z.B. eine separate Scheibe sein, die in die Sinterkammer eingelegt wird. Es ist aber auch denkbar, direkt den Boden der Sinterkammer als diese Basisfläche auszubilden. Die Basisfläche kann zur Ausbildung einer ebenen Gleitfläche aus einem hochtemperaturfesten keramischen Material, wie z.B. Zirkoniumdioxid, Aluminiumoxid oder Siliziumkarbid bestehen. Die Basisfläche kann aber auch aus entsprechend geeigneten Metallen oder Metalllegierungen bestehen. Es kann sich im Sinne von guten Gleiteigenschaften z.B. um eine feingeschliffene, ultrafeingeschliffene oder auch polierte Oberfläche handeln. Die Basisfläche kann selbst durch einen Sintervorgang, gegebenenfalls mit nachgeschaltetem Feinschleifen, Ultrafeinschleifen oder Polieren, entstanden sein. Günstigerweise weist die ebene Gleitfläche einen Mittenrauhwert Ra feiner als 3 µm (Micrometer), vorzugsweise feiner als 0,2 µm, besonders bevorzugt feiner als 0,1 µm, bestimmt nach DIN EN ISO 4287 auf.

Zur Sinterkammer ist noch darauf hinzuweisen, dass dieser Begriff in seiner allgemeinsten Bedeutung aufzufassen ist. Es handelt sich dabei um irgendeine Kammer, in der gesintert werden kann. Dies kann z.B. der Sinterofen selbst sein. Es kann sich aber z.B. auch um eine gesonderte Kammer handeln, die zum Sintern in einen Sinterofen hineingestellt wird. Die Verwendung einer gesonderten Kammer kann z.B. dazu genutzt werden, dass der Sinterrohling in einer in der Sinterkammer erzeugten Schutzgasatmosphäre gesintert wird.

Ein Verfahren zum Sintern eines erfindungsgemäßen Sinterrohlings kann vorsehen, dass der Sinterrohling beim Sintern in einer Sinterkammer mit seiner Gleitnoppe oder seinen Gleitnoppen und soweit vorhanden mit seinem Standfuß oder seinen Standfüßen auf einer als ebene Gleitfläche ausgebildeten Basisfläche in der Sinterkammer zur Ermöglichung eines möglichst verzugsfreien Schrumpfens des Sinterrohlings entlang gleitet.

Ein Verfahren zur Konstruktion und/oder Herstellung eines erfindungsgemäßen Sinterrohlings sieht vor, dass an der Verstrebung zumindest eine Gleitnoppe angeformt wird, mit welcher die Verstrebung während des Sintervorgangs auf der Basisfläche abgestützt werden kann bzw. wird.

Dieses Konstruktions- und/oder Herstellungsverfahren wird bevorzugt unter Verwendung zumindest eines entsprechenden Computerprogramms ausgeführt. Bei einem solchen Computerprogramm handelt es sich bevorzugt um eine Weiterbildung an sich bekannter CAD-Programme, wobei eben bevorzugt bei der Konstruktion des Sinterrohlings an der Verstrebung zumindest eine Gleitnoppe angeformt wird, mit welcher die Verstrebung während des Sintervorgangs auf der Basisfläche abgestützt werden kann. Die Auswahl der Anzahl, Form, Größe und/oder Position der Gleitnoppen auf der Verstrebung kann von diesem Computerprogramm automatisiert durchgeführt werden. Genauso gut ist es möglich, dass der Benutzer des Computerprogramms diese Parameter individuell eingibt. Es ist auch möglich, dass das Computerprogramm einen Vorschlag zu diesen Parametern also der Anzahl, Form, Größe und/oder Position der Gleitnoppen auf der Verstrebung unterbreitet und der Benutzer des Computerprogramms dies dann noch überarbeiten kann. Das Computerprogramm kann bevorzugt auch alle oben genannten bevorzugten Merkmale des Sinterrohlings konstruieren bzw. realisieren. Der vom Computerprogramm konstruierte Sinterrohling liegt im Ergebnis dann günstigerweise in digitaler Form vor. Er wird bevorzugt zur Ansteuerung einer an sich bekannten Einrichtung zur materialabtragenden Bearbeitung eines Blocks aus Ausgangsmaterial verwendet, sodass die Einrichtung zur materialabtragenden Bearbeitung dann anhand des Ergebnisses des Computerprogramms den Sinterrohling aus dem Block aus Ausgangsmaterial materialabtragend herausarbeiten kann. Insofern kann auch eine Einrichtung zur materialabtragenden Bearbeitung eines Blocks aus Ausgangsmaterial vorgesehen sein, wobei vorgesehen ist, dass die Einrichtung den vom Computerprogramm konstruierten erfindungsgemäßen Sinterrohling materialabtragend aus dem Block aus Ausgangsmaterial herausarbeitet. Unter einer materialabtragenden Bearbeitung wird insbesondere ein Fräsen und/oder Schleifen verstanden.

Weitere Merkmale und Einzelheiten bevorzugter Ausgestaltungsformen der Erfindung werden beispielhaft an den schematisiert dargestellten Ausführungsbeispielen erläutert. Es zeigen:
Fig. 1 eine Ansicht von unten auf einen ersten erfindungsgemäßen Sinterrohling;
Fig. 2 und 3 Schnittdarstellungen durch eine Verstrebung im Bereich von verschiedenartig ausgebildeten Gleitnoppen;
Fig. 4 eine schematisiert dargestellte Sinterkammer mit darin angeordneter Basisfläche und darauf aufgestelltem Sinterrohling gemäß Fig. 1;
Fig. 5 und 6 weitere Beispiele erfindungsgemäß ausgebildeter Sinterrohlinge.

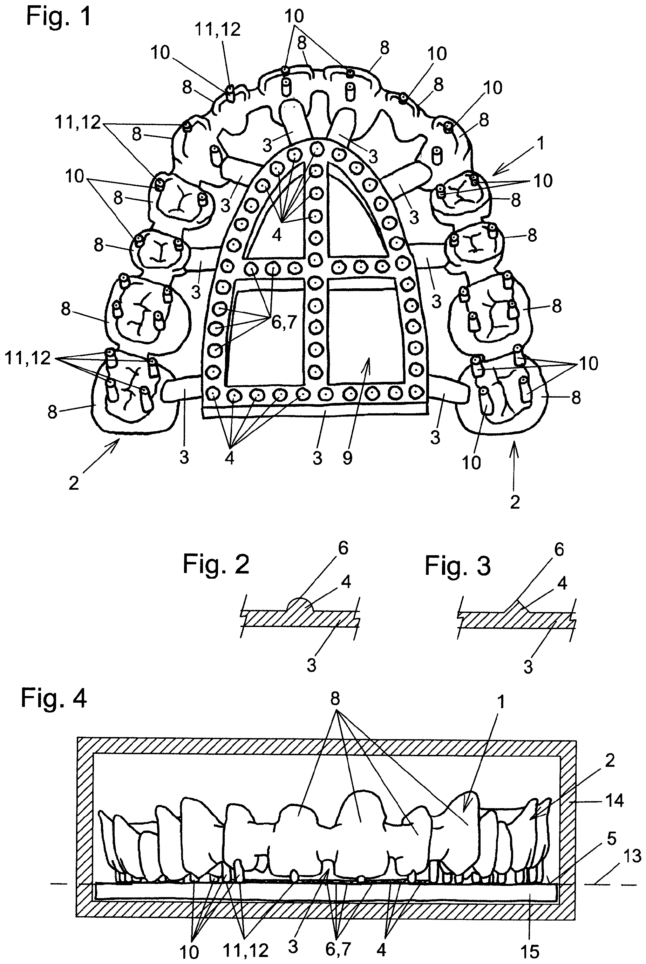

In Fig. 1 ist in einer Ansicht von unten, also von der Unterseite, mit der der Sinterrohling 1 beim Sintern auf der Basisfläche 5 steht, her ein Sinterrohling 1 gezeigt. Der Produktbereich 2, also der Bereich des Sinterrohlings 1, aus dem die Dentalprothese entsteht, ist in diesem Ausführungsbeispiel gemäß Fig. 1 als ein vollständiger Zahnbogen mit einer entsprechenden Anzahl von Zähnen 8 ausgebildet. Die Verstrebung 3 des Sinterrohlings 1 verbindet mehrere Zähne 8 dieses Zahnbogens miteinander, um den Sinterrohling 1 insgesamt stabiler auszugestalten und einen Verzug beim Sintervorgang möglichst zu vermeiden. Erfindungsgemäß weist die Verstrebung 3 Gleitnoppen 4 zum Abstützen der Verstrebung 3 auf der Basisfläche 5 während des Sintervorgangs auf. Jede der Gleitnoppen 4 hat einen Auflagepunkt 6 bzw. eine möglichst kleine Auflagefläche 7, um damit während des Sintervorgangs auf der Basisfläche 5 aufgestellt zu werden. Beispiele für mögliche Ausgestaltungsformen der Gleitnoppen sind in den Fig. 2 und 3 jeweils in einer Schnittdarstellung durch die Verstrebung 3 gezeigt. In Fig. 2 ist die Gleitnoppe 4 halbkugelförmig ausgebildet mit einem punktförmigen Auflagepunkt 6. In Fig. 3 ist beispielhaft eine kegelförmige Gleitnoppe 4 gezeigt. Auch diese weist einen punktförmigen Auflagepunkt 6 auf. Durch entsprechende Abflachung der Gleitnoppen 4 kann natürlich auch eine kreisförmige oder ringförmige Auflagefläche 7 realisiert werden.

In Fig. 1 ist gut zu sehen, dass der hier den Produktbereich 2 bildende Zahnbogen aus den Zähnen 8 einen Innenraum 9 umschließt, in dem die Verstrebung 3 angeordnet ist.

Um auch den Produktbereich 2 direkt auf der Basisfläche 5 abstützen zu können, weist der Sinterrohling 1 im gezeigten Ausführungsbeispiel gemäß Fig. 1 am Produktbereich mehrere voneinander distanziert angeordnete Standfüße 10 auf, mit denen der Produktbereich 2 auf der Basisfläche 5 während des Sintervorgangs abgestützt werden kann. Auch die hier beispielhaft stiftförmig ausgeführten Standfüße 10 weisen möglichst kleine, also Auflagepunkte 11 bzw. möglichst kleine Auflageflächen 12 auf, mit denen sie dann in der Betriebsstellung während des Sintervorgangs auf der Basisfläche 5 abgestützt sind. Nach dem Sintervorgang werden sowohl die Verstrebung 3 als auch die Standfüße 10 durch geeignete, beim Stand der Technik an sich bekannte, Maßnahmen vom Produktbereich 2 entfernt, sodass dann gegebenenfalls mit noch weiteren Nachbearbeitungsschritten die gewünschte Dentalprothese fertiggestellt ist.

In Fig. 4 ist stark schematisiert eine Sinterkammer 14 dargestellt, in der der Sinterrohling 1 aus Fig. 1 in der Betriebsstellung während des Sintervorgangs auf der Basisfläche 5 abgestützt ist. Die Basisfläche 5 ist dabei günstigerweise eine ebene Gleitfläche, wie sie weiter oben detaillierter geschildert ist. Die Gleitnoppen 4 und die Standfüße 10 stützen sich in diesem bevorzugten Ausgestaltungsbeispiel mit ihren Auflagepunkten 6 bzw. 11 oder ihren Auflageflächen 7 bzw. 12 günstigerweise alle auf einer gemeinsamen, durch die entsprechende ebene Gleitfläche gebildete Ebene 13 der Basisfläche 5 ab. Im gezeigten Ausführungsbeispiel ist die Basisfläche 5 eine Oberfläche einer, z.B. keramischen, polierten Scheibe 15, welche in die Sinterkammer 14 eingelegt ist. Es kann aber genauso gut so realisiert werden, dass die Basisfläche 5 direkt von einer entsprechenden Bodenfläche der Sinterkammer 14 ausgebildet wird. Zum grundsätzlichen Aufbau einer Sinterkammer 14 kann auf den Stand der Technik verwiesen werden, sodass hier nicht weiter darauf eingegangen werden muss. Dies gilt insbesondere auch für die verschiedenen Möglichkeiten der Schutzgas- Zu- und Abfuhr in die Sinterkammer 14, um in dieser während des Sintervorgangs eine möglichst oxidfreie Atmosphäre zu schaffen, falls dies von Nöten ist.

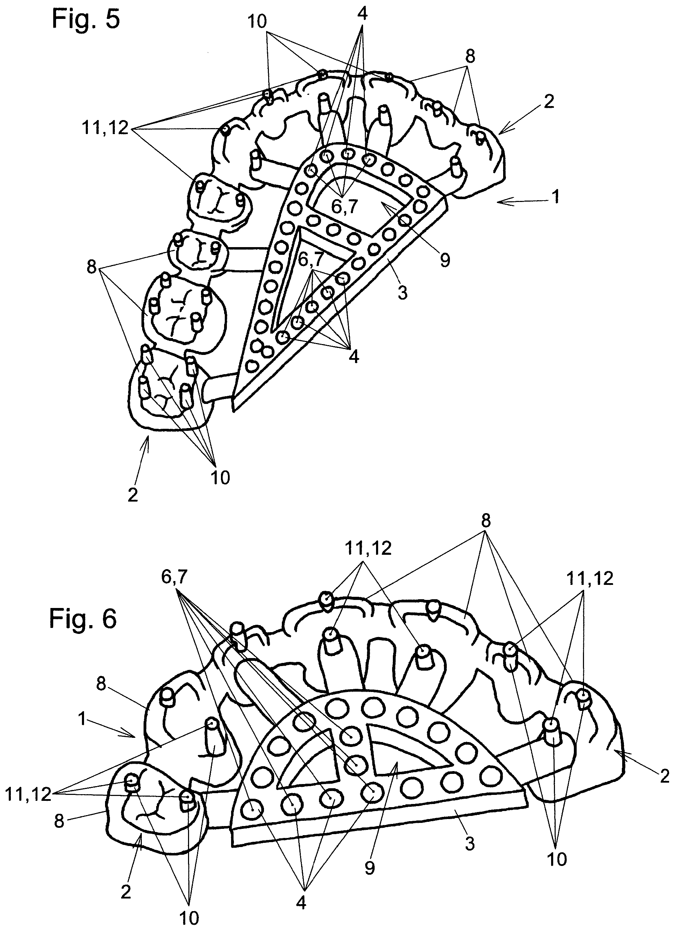

Die Fig. 5 und 6 zeigen beispielhaft weitere Sinterrohlinge, bei denen der Produktbereich 2 auch aus einer Abfolge von Zähnen 8, aber eben nicht aus einem vollständigen Zahnbogen sondern nur aus einem Zahnbogen bestehen, welcher nur einen Teil der natürlichen Zähne des natürlichen Zahnbogens ersetzt. Auch bei diesen Sinterrohlingen 1 ist erfindungsgemäß vorgesehen, dass die Verstrebung 3 entsprechende Gleitnoppen 4 aufweist. Auch am Produktbereich 2 sind entsprechende Standfüße 10 angeordnet. Ansonsten gilt auch für diese Ausführungsbeispiele das zur ersten Variante gemäß Fig. 1 Gesagte.

### Legende zu den Hinweisziffern:

- 1: Sinterrohling
- 2: Produktbereich
- 3: Verstrebung
- 4: Gleitnoppe
- 5: Basisfläche
- 6: Auflagepunkt
- 7: Auflagefläche
- 8: Zahn
- 9: Innenraum
- 10: Standfuß
- 11: Auflagepunkt
- 12: Auflagefläche
- 13: gemeinsame Ebene
- 14: Sinterkammer
- 15: Scheibe

## Patentansprüche

1. Sinterrohling (1) zur Herstellung einer Dentalprothese mit zumindest einem Sintervorgang, wobei der Sinterrohling (1) zumindest einen Produktbereich (2), aus dem die Dentalprothese entsteht, und zumindest eine Verstrebung (3), welche nach dem Sintervorgang zu entfernen ist, aufweist, **dadurch gekennzeichnet, dass** die Verstrebung (3) zumindest eine Gleitnoppe (4) zum Abstützen der Verstrebung (3) auf einer Basisfläche (5) während des Sintervorgangs aufweist, wobei die Gleitnoppe (4) als eine Erhebung auf einer entsprechenden Oberfläche der Verstrebung (3) ausgebildet ist und eine gegenüber der Verstrebung (3) reduzierte Auflagefläche (7) aufweist, so dass die Verstrebung (3) während des Sintervorgangs besser auf der Basisfläche (5) gleiten kann.

2. Sinterrohling (1) nach Anspruch 1, **dadurch gekennzeichnet, dass** die Verstrebung (3) mehrere zueinander distanziert angeordnete Gleitnoppen (4) zum Abstützen der Verstrebung (3) auf der Basisfläche (5) während des Sintervorgangs aufweist.

3. Sinterrohling (1) nach Anspruch 1 oder 2, **dadurch gekennzeichnet, dass** die gegenüber der Verstrebung reduzierte Auflagefläche (7) ein Auflagepunkt (6) ist.

4. Sinterrohling (1) nach einem der Ansprüche 1 bis 3, **dadurch gekennzeichnet, dass** der Produktbereich (2) ein Zahnbogen mit mehreren, vorzugsweise paarweise, untereinander verbundenen Zähnen (8) ist und die Verstrebung (3) zumindest zwei Zähne (8) dieses Zahnbogens miteinander verbindet.

5. Sinterrohling (1) nach Anspruch 4, **dadurch gekennzeichnet, dass** der Zahnbogen einen Innenraum (9) teilweise umschließt und die Verstrebung (3) zumindest teilweise im Innenraum (9) angeordnet ist.

6. Sinterrohling (1) nach einem der Ansprüche 1 bis 5, **dadurch gekennzeichnet, dass** der Sinterrohling (1) am Produktbereich (2) einen Standfuß (10) oder mehrere voneinander distanziert angeordnete Standfüße (10) zum Abstützen des Produktbereichs (2) auf der Basisfläche (5) während des Sintervorgangs aufweist.

7. Sinterrohling (1) nach Anspruch 6, **dadurch gekennzeichnet, dass** der Standfuß (10) oder die Standfüße (10) zum Abstützen des Produktbereichs (2) auf der Basisfläche (5) während des Sintervorgangs, vorzugsweise jeweils, eine gegenüber dem Produktbereich reduzierte Auflagefläche (12), vorzugsweise einen Auflagepunkt (11), aufweist bzw. aufweisen.

8. Sinterrohling (1) nach einem der Ansprüche 1 bis 7, **dadurch gekennzeichnet, dass** alle Auflagepunkte (6, 11) und/oder Auflageflächen (7, 12) aller Gleitnoppen (4) und soweit vorhanden auch aller Standfüße (10) in einer gemeinsamen Ebene (13) angeordnet sind.

9. Sinterkammer (14) zum Sintern zumindest eines Sinterrohlings (1), mit zumindest einem Sinterrohling (1) nach einem der Ansprüche 1 bis 8, **dadurch gekennzeichnet, dass** die Sinterkammer (14) eine ebene Gleitfläche als Basisfläche (5) zum Abstellen des Sinterrohlings (1) auf dieser Basisfläche (5) während des Sintervorgangs aufweist.

## Claims

1. A sinter blank (1) for producing a dental prosthesis in at least one sintering process, the sinter blank (1) having at least one product area (2), from which the dental prosthesis is obtained, and at least one strut (3), which is to be removed after the sintering process, **characterized in that** the strut (3) has at least one sliding knob (4) for supporting the strut (3) on a base surface (5) during the sintering process, wherein the sliding knob (4) is designed as an elevation on a corresponding surface of the strut (3) and has a reduced bearing surface (7) in relation to the strut (3), so that the strut (3) can better slide on the base surface (5) during the sintering process.

2. The sinter blank (1) as claimed in claim 1, **characterized in that** the strut (3) has a plurality of sliding knobs (4) arranged at a distance from one another for supporting the strut (3) on the base surface (5) during the sintering process.

3. The sinter blank (1) as claimed in claim 1 or 2, **characterized in that** the bearing surface (7), which is reduced in relation to the strut, is a bearing point (6).

4. The sinter blank (1) as claimed in one of claims 1 through 3, **characterized in that** the product area (2) is a dental arch with a plurality of teeth (8) interconnected, preferably in pairs, and the strut (3) connects at least two teeth (8) of this dental arch to each other.

5. The sinter blank (1) as claimed in claim 4, **characterized in that** the dental arch partially encloses an interior (9), and the strut (3) is arranged at least partially in the interior (9).

6. The sinter blank (1) as claimed in one of claims 1 through 5, **characterized in that**, on the product area (2), the sinter blank (1) has a support foot (10), or a plurality of support feet (10) arranged at a distance from one another, for supporting the product area (2) on the base surface (5) during the sintering process.

7. The sinter blank (1) as claimed in claim 6, **characterized in that** the support foot (10) or the support feet (10) for supporting the product area (2) on the base surface (5) during the sintering process has or have, preferably in each case, a reduced bearing surface (12) in relation to the product area, preferably a bearing point (11).

8. The sinter blank (1) as claimed in one of claims 1 through 7, **characterized in that** all the bearing points (6, 11) and/or bearing surfaces (7, 12) of all the sliding knobs (4) and also of all the support feet (10), if the latter are present, are arranged in a common plane (13).

9. A sintering chamber (14) for sintering at least one sinter blank (1) with at least one sinter blank (1) as claimed in one of claims 1 through 8, **characterized in that** the sintering chamber (14) has a plane sliding surface as base surface (5) on which to place the sinter blank (1) during the sintering process.

## Revendications

1. Ébauche à fritter (1) pour la fabrication d'une prothèse dentaire avec au moins un processus de frittage, l'ébauche à fritter (1) présentant au moins une zone de produit (2) à partir de laquelle est fabriquée la prothèse dentaire et au moins un entretoisement (3) à retirer après le processus de frittage, **caractérisée en ce que** l'entretoisement (3) présente au moins un plot de glissement (4) pour appuyer l'entretoisement (3) sur une surface de base (5) pendant le processus de frittage, le plot de glissement (4) étant formé comme une partie surélevée sur une surface correspondante de l'entretoisement (3) et présentant une surface d'appui (7) réduite par rapport à l'entretoisement (3), de sorte que l'entretoisement (3) peut mieux glisser sur la surface de base (5) pendant le processus de frittage.

2. Ébauche à fritter (1) selon la revendication 1, **caractérisée en ce que** l'entretoisement (3) présente une pluralité de plots de glissement (4) espacés les uns des autres pour appuyer l'entretoisement (3) sur la surface de base (5) pendant le processus de frittage.

3. Ébauche à fritter (1) selon la revendication 1 ou 2, **caractérisée en ce que** la surface d'appui (7), qui est réduite par rapport à l'entretoisement, est un point d'appui (6).

4. Ébauche à fritter (1) selon l'une des revendications 1 à 3, **caractérisée en ce que** la zone de produit (2) est un arc dentaire avec plusieurs dents (8) avec plusieurs dents (8) reliées entre elles de préférence par paires, et l'entretoisement (3) relie entre elles au moins deux dents (8) de cet arc dentaire.

5. Ébauche à fritter (1) selon la revendication 4, **caractérisée en ce que** l'arc dentaire entoure partiellement un espace intérieur (9) et l'entretoisement (3) est disposé au moins partiellement dans l'espace intérieur (9).

6. Ébauche à fritter (1) selon l'une des revendications 1 à 5, **caractérisée en ce que** l'ébauche à fritter (1) présente sur la zone de produit (2) un pied support (10) ou plusieurs pieds support (10) espacés les uns des autres pour appuyer la zone de produit (2) sur la surface de base (5) pendant le processus de frittage.

7. Ébauche à fritter (1) selon la revendication 6, **caractérisée en ce que** le ou les pieds d'appui (10) pour appuyer la zone de produit (2) sur la surface de base (5) pendant le processus de frittage, présente ou présentent, de préférence chacun, une surface d'appui (12), de préférence un point d'appui (11), qui est réduite par rapport à la zone de produit.

8. Ébauche à fritter (1) selon l'une des revendications 1 à 7, **caractérisée en ce que** tous les points d'appui (6, 11) et/ou toutes les surfaces d'appui (7, 12) de tous les plots de glissement (4) et, dans la mesure où il y en a, également tous les pieds d'appui (10) sont disposés dans un même plan (13).

9. Chambre de frittage (14) pour le frittage d'au moins une ébauche à fritter (1), avec au moins une ébauche à fritter (1) selon l'une des revendications 1 à 8, **caractérisée en ce que** la chambre de frittage (14) présente une surface de glissement plane en tant que surface de base (5) pour poser l'ébauche à fritter (1) sur cette surface de base (5) pendant le processus de frittage.
